Europäisches Patentamt

**European Patent Office**

Office européen des brevets

(19)

(11) Publication number: **0 356 397**
**A2**

(12)

# EUROPEAN PATENT APPLICATION

(21) Application number: **89810618.2**

(22) Date of filing: **21.08.89**

(51) Int. Cl.⁵: **C 12 P 21/08**
**C 12 N 15/02, A 61 K 39/00,**
**A 61 K 39/395**

(30) Priority: **24.08.88 GB 8820036**

(43) Date of publication of application:
**28.02.90 Bulletin 90/09**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Stahel, Rolf A.**
**Höhenstrasse 48**
**CH-8127 Forch (CH)**

**Waibel, Robert**
**Räterschenstrasse 5**
**CH-8352 Ricketwil (CH)**

The applicant has filed a statement in accordance with Rule 28 (4) EPC (issue of a sample only to an expert). Accession number(s) of the deposit(s): ECACC 88080301 (see encl.)

(54) **Monoclonal antibodies against, and cell surface antigen of, lung carcinoma.**

(57) Monoclonal antibodies and fragments thereof, hybrid cell lines producing these antibodies and methods of using them are described. They are characterized in that they
- belong to Cluster w4 as defined at the International Workshop on SCC, London 1987,
- show high capacity for in vivo localisation,
- have low retention in non-target organs,
- exhibit persistence at the target tumor and
- bind to a glycoprotein antigen having the following characteristics:
- molecular weight of approximately 45000 before and about 15000 to about 17000 after deglycosylation;
- remains reactive with antibody SWA11 after deglycosylation;
- remains present in cells after treatment with tunicamycin.
They have an interesting pharmacological activity and may thus find application in the diagnosis and/or treatment of lung carcinoma.

Description

## MONOCLONAL ANTIBODIES AGAINST, AND CELL SURFACE ANTIGEN OF, LUNG CARCINOMA

### FIELD

The invention is directed to monoclonal antibodies against an antigen of the small cell carcinoma of the lung, hybrid cell lines producing these antibodies and methods of using these monoclonal antibodies.

### BACKGROUND

Lung cancer is currently the most lethal of malignant conditions in the Western world and is predicted to remain so for the foreseeable future. Of the five major groups of lung cancer only small cell cancer (SCC) is observed to be highly chemo- and radiosensitive and yet the prognosis for patients with SCC remains very poor. Approximately 60 % of SCC patients have metastases at the time of first presentation, restricting the use of surgery or radiotherapy and causing a strong reliance on the use of chemotherapy. The poor prognosis for SCC relates not only to its early and extensive metastasis but to its high rate of growth and the post-treatment emergence of chemoresistance.

This invasive nature indicates the necessity to develop new agents with systemic efficacy against SCC.

On the other hand the use of monoclonal antibodies (MAbs) for diagnosis, imaging and now even therapy of malignant disease is an increasingly realistic objective. The treatment of SCC using MAbs, i.e. by immunotherapy, e.g. by radioimmunotherapy, appears as a particularly attractive proposition.

Unfortunately there is a great scarcity of suitable monoclonal antibodies for these applications. Thus, e.g. for radioimmunotherapy, there is a need for monoclonal antibodies with more desirable properties than for those which are currently available, such as higher tumor-to-blood and tumor-to-tissue ratios. These shortcomings are also evidenced by the limited success achieved so far in the radioimmunotherapeutic treatment of human tumor xenografts grown in rodent models. In one study complete ablation of a radiosensitive neuroblastoma has been reported but other cases show a high dependency on the age of the tumor, with only limited inhibition of growth in those which are well established.

The MAbs generated against surface antigens of SCC can be divided according to the nature and expression of their target antigens and other immunological characteristics. Five such groups named Clusters have been defined at the International Workshop on SCC, London 1987 (The Lancet [August 8, 1987] 325). One such cluster is Cluster w4.

### SUMMARY

The invention provides monoclonal antibodies, e.g. of IgG isotype which are suitable for use in the diagnosis and immunotherapy of the small cell carcinoma of the lung and belong to Cluster w4 as defined at the International Workshop on SCC, London 1987.

A further object of the invention is to provide methods for the diagnosis and treatment of small cell carcinoma of the lung using monoclonal antibodies, e.g. of the IgG isotype, which belong to Cluster w4, e.g methods for suppressing small cell carcinoma of the lung in an animal using such antibodies.

The invention further includes hybrid cell lines which produce these MAbs as well as methods for using and processes for preparing these MAbs.

The invention further provides a novel antigen to which the above monoclonal antibodies bind.

The new MAbs of the invention are characterized by a very high capacity for in vivo localisation, relatively low retention in non-target organs and persistence at the target tumour. These properties make them suitable in diagnostic and, especially, in therapeutic applications as vehicles for e.g. the selective delivery of cytotoxins or isotopes for imaging or therapy of SCC.

Preferred are the monoclonal antibodies SWA11, SWA21 and SWA22, especially SWA11.

The ability of the monoclonal antibodies to react with small cell carcinoma of the lung whilst having restricted reactivity with non-small cell carcinoma and normal tissues is very significant in terms of the detection of these tumors and the immunotherapeutic use of these monoclonal antibodies.

### DESCRIPTION OF THE PREFERRED EMBODIMENTS

The invention relates to monoclonal antibodies of preferably IgG, e.g. IgG2a, IgG2b or IgG3 isotype, which belong to Cluster w4 as defined at the International Workshop on SCC, London 1987. The MAbs recognize a tumour-associated antigen which is selectively expressed on the small cell carcinoma of the lung and which show little expression on other pulmonary and non-pulmonary cancers and normal tissue, and which appears not to correspond to any other antigen known. Some antibody reactivity is observed with about 5 % of the total white cell population of human peripheral blood buffy coat. This reactivity is restricted to about 40 % of mature granulocytes.

The general methods for the production of hybridomas which secrete such monoclonal antibodies is well known to those of ordinary skill in the art. Illustrative of the techniques employed are those described in Cancer Res. 46 (1986) 2077. Briefly, BALB-C mice are immunised with viable cells of the SW2 small cell carcinoma cell line. After sacrifice of the animals, the spleen cells are fused with the NS-1 myeloma cell line and the hybridomas screened for their ability to produce antibodies using the SW2 cell line.

Further screening, including determination of high capacity for in vivo localisation, low retention in non-target organs and persistance at the target tumour in accordance with standard methods led to the selection of a group of hybridoma cell lines secreting the monoclonal antibodies belonging to Clusterw4, particularly SWA11, SWA21 and SWA22.

A first subgroup, including e.g. SWA11, is characterized by a high affinity for SW2 target cells with an association constant $K_a$ of about $1 \times 10^9$ $M^{-1}$ or higher, as evidenced by a very strong localization to xenografts grown in nude mice.

A second subgroup, including e.g. SWA21 and SWA22, is characterized by a lesser degree of affinity for SW2 target cells with an affinity constant $K_a$ of between about $10^7$ and about $10^9$ $M^{-1}$.

The hybridoma cell line secreting SWA11 has been deposited on August 3, 1988 under Deposit Number 88080301 with the European Collection of Anmimal Cell Cultures (ECACC) in Porton Down, U.K. under the provisions of the Budapest Treaty.

The above-mentionned further screening is explained in more detail in the Examples. It has shown that the MAbs of the invention, particularly SWA11, are exceptionally well-suited for use in the diagnosis and especially in the therapy of SCC, in view of, i.a.:
- an exceptional pattern of cell line reactivity: thus, SWA-11 reacts with all the SCC cell lines tested so far;
- remarkable tissue specificity for SCC tumors in vivo (nude mice xenografts);
- high binding affinity;
- persistance at the target tumor.

The MAbs of the invention may be used essentially according to methods known in the art.

They may for example be derivatised.

It is thus foreseen that anti-idiotype antibodies may be produced, such techniques being within the capabilities of a person of ordinary skill in the art. Briefly, an anti-idiotype antibody is an antibody which recognises unique determinants present on the antibody of interest. Thus a monoclonal antibody such as SWA11 is injected into an animal such as was used as the source of the original monoclonal antibody producing cell. The animal immunised will recognise and respond to the idiotypic determinants of the immunising antibody by producing an antibody to these idiotypic determinants (the anti-idiotypic antibody). These anti-idiotypic antibodies, which are specific for a monoclonal antibody itself specific for the particular antigen of interest, will, if injected into a further host, stimulate the production of anti-(anti-idiotypic) antibodies. The anti-(anti-idiotypic) antibodies will be of the same specificity as the original monoclonal antibody and will themselves identify the antigen of interest. The advantage of employing such a system is that the antibodies produced in the final host which bears the antigen of interest, are endogenous to that host and may be produced naturally in vast quantities. Also, only very small amounts of anti-idiotype antibody need be administered to the final host, such amounts being sufficient only to stimulate an immune response.

Anti-idiotype antibodies may also be used in the isolation of other hybridoma cell lines secreting monoclonal antibodies having the specificity of the monoclonal antibodies of the invention. Such anti-idiotype screening may readily be accomplished by one of ordinary skill in the art. Briefly, anti-idiotype antibodies are produced as described before, and by using these anti-idiotype antibodies, which are specific for the monoclonal antibodies produced by a single hybridoma, it is possible to identify other clones with exactly the same idiotype as the antibody of the hybridoma used for immunisation. Idiotypic identity between monoclonal antibodies of two hybridomas demonstrates that the two monoclonal antibodies are the same with respect to their recognition of the same epitopic determionant. Thus, by using antibodies to the epitopic determinants on a monoclonal antibody it is possible to identify other hybridomas expressing monoclonal antibodies of the same epitopic specificity. In antibodies, these idiotypic determinants are present in the hypervariable region which binds to a given epitope.

While the use of foreign donor monoclonal antibody of one species in a second recipient species is usually not a factor in in vivo immunotherapy or immunodiagnosis, a potential problem which may arise is the occurrence of an adverse immunological response by the host to antigenic determinants present on the donor antibody. In some instances, this adverse response can be so severe as to curtail the in vivo use of the donor antibody in the host. Further, this adverse host response may serve to hinder the malignancy-suppressing efficacy of the donor antibody. One way in which it is possible to cirumvent the likelihood of an adverse immune response occuring in the host is by using chimeric antibodies (Hybridoma 5, S17 [1986] and Biotechniques 4, 214 [1986]). Chimeric antibodies are antibodies in which the various domains of the antibody heavy and light chains are coded for by DNA from more than one species. Typically, a chimeric antibody will comprise the variable domains of the heavy ($V_H$) and light ($V_L$) chains derived from the donor species producing the antibody of desirable antigenic specificity and the constant antibody domains of the heavy ($_{CH}$) and light ($C_L$) chains derived from the host recipient species. It is believed that by reducing the exposure of the host immune system to the antigenic determinants on the donor antibody domains the possibility of an adverse immunological response occuring in the recipient species will be reduced. Thus, for example, it is possible to produce a chimeric antibody for in vivo clinical use in humans which comprises mouse $V_H$ and $V_L$ domains coded for by DNA isolated from the SWA11-producing cell line and $C_H$ and $C_L$ domains coded for by DNA isolated from a human cell.

Under certain circumstances, monoclonal antibodies of a particular class may be preferable, in terms of diagnostic or therpeutic efficacy, over those of a different class. For example problems may be associated with the utilisation of antibodies of the IgM class, such including high cross-reactivity in tissue staining, poor tissue penetration and difficulties with quantitative biochemical manipulations such as the production of immunoconjugates. Similarly, monoclonal antibodies of a particular isotype might be preferable to those of another. In this respect it is known that mouse monoclonal antibodies of isotype gamma-2a, gamma-2b and gamma-3 are more effective in inhibiting the growth of tumors than is the gamma-1 isotype. This differential efficacy is thought to be due to the ability of these isotypes to participate more actively in cytolytic destruction of tumour cells. Particular isotypes of a monoclonal antibody can be prepared either directly by selecting from the initial hybridoma fusion or prepared secondarily from a parental hybridoma secreting a monoclonal antibody of different isotype and selecting for class-switch variants having the same specificity as, for example, SWA11.

The monoclonal antibodies of the invention or anti-idiotypic or chimeric antibodies having the same specificity or stimulating the production of antibodies with the same specificity respectively, can be used in any animal in which it is desirable to administer in vitro or in vivo immunodiagnosis or immunotherapy. The term "animal" as used herein is meant to include both humans as well as non-humans.

The term "antibody" as used herein is meant to include intact molecule as well as fragments thereof, such as, for example, Fab and F(ab')2, which are capable of binding the epitopic determinant.

The term "essentially non-reactive" when used to characterise the reactivity between the monoclonal antibodies of the iinvention and an antigen means that any reaction which might occur is insignificant in terms of limiting the diagnostic or therapeutic usefulness of the antibodies.

The monoclonal antibodies of the invention are also suited for use in immunoassays in which they can be used in liquid phase or bound to a solid phase carrier. In addition, the monoclonal antibodies in these immunoassays can be detectably labeled in various ways. Examples of types of immunoassay which can use the monoclonal antibodies of the invention are competitive and non-competitive immunoassays, common examples being the radioimmunoassay (RIA) and the enzyme-linked immunosorbent assay (ELISA). There are many different labels and methods of labeling for the monoclonal antibodies, and those of ordinary skill in the art will either know of such or will be able to ascertain such using routine experimentation, and will be able to effect binding using standard techniques.

In using the monoclonal antibodies of the invention for the in vivo detection of antigen, the detectably labeled monoclonal antibody is given in a dose which is diagnostically effective. The term "diagnostically effective" means that the amount of detectably labeled monoclonal antibody is administered in sufficient quantity to enable detection of the site having the small cell carcinoma antigens for which the monoclonal antibodies are specific. The concentration of detectably labeled monoclonal antibody which is administered should be sufficient such that the binding to the tumour site is detectable compared to the background signal. Further, it is desirable that the detectably labeled monoclonal antibody be rapidly cleared from the circulatory system in order to give the best tumour-to-background signal ratio.

As a rule the dosage of detectably labeled monoclonal antibody for diagnosis will vary depending on such factors as the age, sex and extent of disease of the individual. The dosage of monoclonal antibody can vary from about 10 mg to about 10 g, preferably from about 10 mg to about 1 g.

The monoclonal antibodies of the invention can also be used, alone or in combination with therapeutic agents for immunotherapy in an animal. These agents can be coupled either directly or indirectly to the monoclonal antibodies of the invention. Examples of therapeutic agents which can be coupled to the monoclonal antibodies of the invention for immunotherapy are e.g. drugs, radioisotopes, immunomodulators, lectins or toxins.

For example, ricin is a toxic lectin which has been used immunotherapeutically and this is accomplished by binding the $\alpha$-peptide chain of ricin, which is responsible for its toxicity, to the antibody molecule to enable site-specific delivery of the toxic effect. In a similar manner toxins such as the diphteria toxin fragment A produced by the microorganism Corynebacterium diphtheriae may be linked to the antibody and used for site-specific delivery of the toxin molecule. Such toxin fragments may be linked to the antibody using linker molecules or similar techniques, or may otherwise be produced by a gene fusion and expression of the hybrid protein directly.

In preliminary studies the antibody SWA11 has been tested for its ability to selectively kill small cell carcinoma cells after conjugation to two forms of the plant toxin ricin.

In a first study the antibody was conjugated to the A-chain of ricin, thereby producing an immunoconjugate capable of killing tumor cells in vitro with an $LC_{50}$ (lethal concentration 50: the concentration of immunoconjugate resulting in 50 % killing of target cells) of $4 \times 10^{-10}$ M, whereas a control conjugate showed an $LC_{50}$ of $> 1 \times 10^{-7}$M. Importantly, in this test system it was apparent that essentially all cells were susceptible to the immunoconjugate.

In a second study the antibody SWA11 was linked to the whole ricin molecule after chemical blockade of the toxin binding site. In this form the SWA11 immunoconjugate yielded an $LC_{50}$ of $7 \times 10^{-12}$ M, whereas the control conjugate gave an $LC_{50}$ of around $1 \times 10^{-8}$ M.

Other therapeutic agents which can be coupled to the monoclonal antibodies of the invention are known, or can readily be ascertained by those of ordinary skill in the art.

The dosage ranges for the administration of the monoclonal antibodies of the invention are those large enough to produce the desired effect in which the symptoms of the small cell carcinoma are ameliorated. The dosage should not be so large as to cause adverse side effects, such as unwanted cross-reactions, anaphylactic reactions and the like. Generally, the dosage will vary with the age, condition, sex and the extent

4

of the disease in the patient and can be determined by one of ordinary skill in the art. Dosage can vary from about 10 mg to about 10 g, preferably from about 10 mg to about 1 g. The antibodies can be administered parenterally by injection or by gradual perfusion over time. The monoclonal antibodies of the invention can be administered intravenously, intraperitoneally, intramuscularly, subcutaneously, intracavity or transdermally, alone or in combination with effector cells.

The invention thus also comprises a tumor-associated antigen selectively expressed on the cell surface membrane of a proportion of small cell carcinoma of the lung, characterized in that it is a glycoprotein which
- has a molecular weight of approximately 45000 before and about 15000 to about 17000 after deglycosylation;
- remains reactive with antibody SWA11 after deglycosylation;
- remains present in cells after treatment with tunicamycin.

The above characterising aspects are examined in more detail in Example 5.

The invention also comprises a pharmaceutical composition comprising an amount of a monoclonal antibody as defined above sufficient to suppress small cell carcinoma of the lung, and a pharmaceutical carrier therefor.

It also comprises a monoclonal antibody as defined above for use as a pharmaceutical, in particular for use in the suppression of small cell carcinoma of the lung.

It also comprises a method of detecting small cell carcinoma of the lung which comprises contacting a source suspected of having SCC of the lung with a diagnostically effective amount of a detectably labelled monoclonal antibody as defined above and determining whether said antibody binds to said source.

It further comprises a method of suppressing small cell carcinoma of the lung which comprises administering to a subject in need of such treatment a therapeutically effective amount of a monoclonal antibody as defined above.

It further comprises a process for the preparation of a pharmaceutical composition comprising mixing a monoclonal antibody as defined above and a pharmaceutical carrier therefor.

The antigen of the invention, $sGP_{45}$, was characterized as follows:

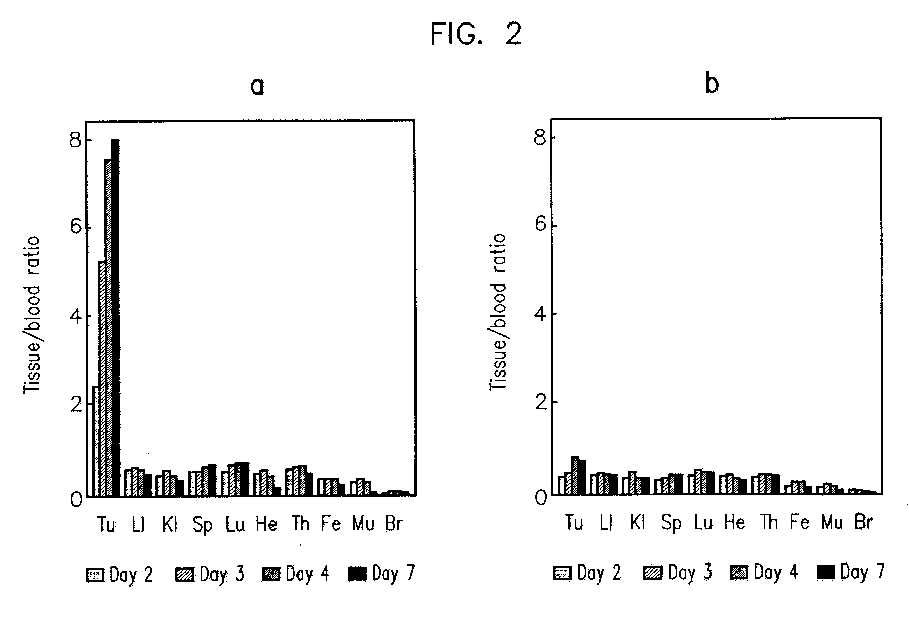

In an in vivo analysis of the uptake of one monoclonal antibody of the invention, the antibody SWA11 was labeled with $^{125}I$ by the Iodogen method and was found to retain a biological activity of around 70 %. When injected intravenously into nude mice bearing SW2 xenografts, SWA11 was found to localize preferentially to the heterotransplants, achieving a level eight fold higher than a control immunoglobulin. The absolute value of localizing SWA11 was 11 % of the injected dose/g of tumor. Thus, the selective uptake of antibody in small cell carcinoma heterotransplants in nude mice was demonstrated. Figure 2a and 2b show the localization of labeled monoclonal antibody SWA11 in various tissues after such an administration, in comparaison with normal labeld IgG. It may readily be seen that the largest percentage of the SWA11 is to be found in the tumor tissue.

The antigen of the invention, $sGP_{45}$, is preferentially expressed on the cell surface of small cell cancer of the lung. Examination of the reactivity of SWA11 with sections of frozen tissues showed the antibody to stain homogenously all seven small cell carcinomas tested and to react with some renal, stomach, colon, breast and pancreatic tumors. Some heterogenous reactivity was also observed with normal tissues originating in colon, bile, duct, skin and thyroid.

The indirect immunofluorescence staining indicated that $sGP_{45}$ is located on the cell surface membrane of cells of small cell carcinoma of the lung and experiments were performed to determine with which fraction of the membrane the antigen is associated. No antigen was observed in crude glycolipid extracts using thin layer chromatography on silica gels. Electrophoresis of small cell carcinoma cell-extracts on 15 % SDS-polyacrylamide gels, under reducing conditions using the buffer system of O'Farrel, renaturing the proteins in the gels in 6 M urea, 3 M urea and transfer buffer (R. Waibel et al., Cancer Res. 47 [1987] 3766-3770), followed by transfer of proteins, electrophoretically, onto 0.2 μm nitrocellulose according to the method of Otter (Anal. Biochem. 162 [1987] 370) in a two-step process beginning with the elution of low molecular weight molecules at low current density followed by prolonged electrotransfer at high current density and incubation with the antibody supernatant and affinity-purified alkaline phosphatase-conjugated goat anti-mouse IgG allowed the detection of antigen in the protein component of the small cell carcinoma cell membrane. The presence of antigen was demonstrated by the measurement of alkaline phosphatase activity using the color development reagents BCIP (5-bromo-4-chloro-3-indolyl phosphate p-toluidine) and NBT (Nitrotetrazolinium Blue chloride) as substrates.

The antigen $sGP_{45}$ has been demonstrated, by biochemical characterization, to be a glycoprotein. Such techniques include deglycosylation of the isolated antigen with the enzyme neuraminidase, chemical deglycosylation of the antigen with periodate or trifluoromethane sulphonic acid, and determining whether the antibody remains reactive with an antigen so treated. The antigenicity was still intact after these treatments.

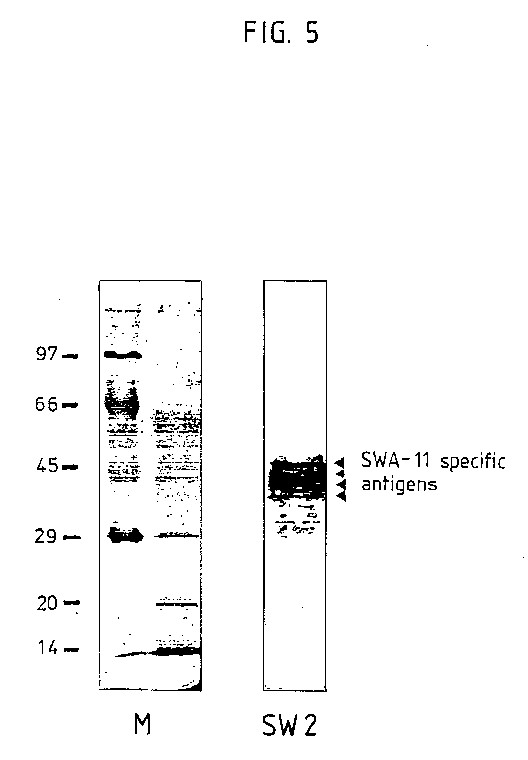

As illustrated in Figure 5 the antigen sGP45, under renaturating immunoblotting techniques using the monoclonal antibody SWA11, produces several molecular weight bands at approximately MW 45000.

Chemical deglycosylation of $sGP_{45}$ with trifluoromethane sulfonic acid produced an antigen of lower molecular weight (MW 15000-17000).

To demonstrate the nature of the antigen, SW2 cells were cultivated in medium containing 10 μg/ml tunicamycin. The antibiotic prevents the formation of glucosamine (Glc) - Asn (asparagine) -linked sugar units. The antigen recognized by SWA11 was still present on tunicamycin-treated cells, whereas the antigen $sGP_{100}$ (SWA20) (EP 323802) and the antigen $sGP_{90-135}$ (LAM8) were completely lost, as demonstrated with indirect immunofluorescence on live cells. These findings clearly demonstrate that the antigen recognized by SWA11 is different from earlier described antigen such as $sGP_{100}$ and $sGP_{90-135}$.

It will be appreciated that the isolation and characterization of the antigen with which for example SWA11 is reactive, enables the possibility of virtually unlimited generation of additional monoclonal antibodies reactive with that antigen. This may be effected according to methods known in the art.

The antigen itself may also find application in the therapy of small cell carcinoma, e.g. in the production of appropriate vaccines.

The above disclosure generally describes the present invention. A more complete understanding can be obtained by reference to the specific Examples hereunder, which are provided herein for purposes of illustration only, and are not intended to limit the scope of the invention.

## DESCRIPTION OF THE DRAWINGS

**Figure 1**: Estimation of n and $K_a$ values for SWA11: graph showing the determination of the number of SWA11 binding sites on $SW_2$ cells

$$( \; \text{---} \; \text{\Large *} \; \text{---} \; ) ,$$

K562 cells

$$( \cdots \cdots \; \boxdot \; \cdots \cdots )$$

and on human peripheral blood buffy coat (----o----) and of the respective association constants of their interactions.

**Figures 2 a) and b)**: Diagram showing the tissue-to-blood ratios for antibody SWA11 (a) and control anti-CEA (anti-carcioembryonic antigen) antibody (b) at 2, 3, 4 and 7 days after i.v. injection of 10 μCi radiolabeled material. Tu= tumour; Li= liver; Ki= kidney; Sp= spleen; Lu= lung; He= heart; Th= thyroid; Fe= femur; Mu= muscle; Br= brain.

**Figure 3 a) and b)**: Diagram showing gamma scintigraphy following i.v. injection of 100 μCi $^{131}$I-labelled SWA11: a) is 2 days post injection, b) is 4 days post injection into nude mice bearing SW2 xenografts.

**Figure 4**: Effect of radiolabeled SWA11 on tumor growth: graph showing the growth rate of SW2 xenografts following i.v. injection of SWA11 alone (...□...), $^{131}$I-labelled SWA11

$$( 300 \; \mu\text{Ci:} \; \text{---}\triangle\text{---}; \; 600 \; \mu\text{Ci:} \; \text{-..-}\blacksquare\text{-..-} ) ,$$

$^{131}$I-labelled anti-CEA (300 μCi; ---0---) or diluent alone (---*---).

**Figure 5**: Immunoblot of SWA11 showing the molecular weights of an N-dodecyl-N,N-dimethylammonio-3-propanesulfonate (sulfobetaine 12) extract of SW2 small cell carcinoma of the lung cells, renatured after PAGE with urea (= Lane SW2) (Lane M = molecular weight markers in kd).

## Example 1: Production and purification of monoclonal antibodies SWA11, SWA21 and SWA22

The SCC cell line SW2 was routinely grown in RPMI medium supplemented with 1 mM glutamine and 10 % foetal calf serum. The antibodies SWA11, -21 and -22 were produced as has been previously described [Stahel et al., Int. J. Cancer 35 (1985) 11].

The antibodies were then purified as follows: a 30-55 % ammonium sulphate fraction was taken from culture supernatant and adsorbed onto a protein A column in PBS. The adsorbed IgG was eluted with 100 mM citrate buffer (pH 4.5) and then dialysed against 10 mM phosphate buffer (pH 6.8) containing 0.01 mM $CaCl_2$. The antibody was then applied to a hydroxylapatite column (Bio-Gel HPHT, Bio-Rad, Richmond, CA.) and then eluted with a linear gradient to 350 mM phosphate.

## Example 2: In vitro characterization

### 1. Reactivity with viable cells by immunofluorescence and haemagglutination

The reactivity of SWA11, SWA21 and SWA22 with a panel of in vitro SCC lines, primary cultures of bronchial epithelium and peripheral white blood cells was determined by indirect immunofluorescence. Cells were washed 3x in phosphate-buffered saline (PBS) (1 % bovine serum albumin, 0.02 % azide), dispensed at $10^5$ cells per tube and incubated for 30 min at 37°C with 100 μl culture supernatant. After further washing the cells were incubated with 50 μl goat anti-mouse fluorescein isothiocyanate (FITC) conjugate (1/20 dilution) for 30 min at 37°C. Rim pattern fluorescence, indicative of surface antigen, was viewed using a Zeiss epifluorescence microscope. Reactivity with a panel of red blood cells expressing defined blood group antigens was assessed by standard direct haemagglutination assay. Table I shows the reactivity of antibodies SWA11, -21 and -22 with cell lines and normal tissue:

## Table I

### Antibody reactivity with cell lines and normal tissue by indirect immunofluorescence and radioimmunoassay

| Cell line | SWA 11 | 21 | 22 |
|---|---|---|---|
| Small cell carcinoma (lung) | | | |
| SW2 | + | + | + |
| OH1 | + | + | + |
| OH3 | + | + | + |
| H69 | + | + | + |
| H128 | + | + | + |
| H60 | + | + | + |
| CORL47 | + | + | + |
| ZL2 | + | + | + |
| Adenocarcinoma (lung) | | | |
| SLC52 | – | – | – |
| A549 | + | + | + |
| A427 | – | – | – |
| CaLu6 | – | – | – |
| Sqamous (lung) | | | |
| U1752 | – | – | – |
| Hotz | + | + | + |
| SK–Mes–1 | – | – | – |
| Large cell (lung) | | | |
| SLC6 | – | – | – |
| ZL23 | – | – | – |
| Mesothelioma (lung) | | | |
| SPC78 | – | – | – |
| SPC212 | – | – | – |

7

(Table I [cont.]

Leukaemic

| | | | | |
|---|---|---|---|---|
| | K562 | + | + | + |
| | CEM | – | – | – |
| | U937 | – | – | – |
| Normal tissue | | | | |
| | Bone marrow | +/– | +/– | +/– |
| | Peripheral blood | +/– | +/– | +/– |
| | Bronchial epithelium | – | – | – |

+/– = small proportion of cells showing heterogenous staining

It appears from Table I that by indirect immunofluorescence SWA11 was seen to react with all 9 small cell carcinoma lines examined. With other lung-derived lines reactivity was seen in 1 of 4 lines of adenocarcinoma origin, 1 of 3 squamous, 0 of 2 large cell and 0 of 2 mesothelial. Reactivity was also seen with one of three leukaemic cell lines. A clear homology was seen between all three antibodies in terms of pattern of cell line reactivity.

Antibody SWA11 was unreactive with primary cultures of normal bronchial epithelial cells but against normal human peripheral blood buffy coat and bone marrow heterogenous staining was seen and fluorescence-activitated cell sorter analysis on the buffy coat has indicated this to be due to cross reactivity with 5 % of the total white cell population. This reactivity is confined to about 40 % of mature granulocytes. By agglutination assay no reactivity of SWA11 was detected against cells bearing the red blood cells A1, A2, B, O, Rh-hr, Kell, Duffy, Kidd, X-linked, Lewis, MNS or P antigens.

The response of antibodies SWA21 and SWA22 was essentially similar.

2. Radioimmunoassay

2.1. Radiolabeling

In all cases the SWA11 antibody was labeled with radioactive iodine by the iodogen technique. Iodogen 0.1 mg was dissolved in 0.2 ml chloroform and added to a 1 ml vial. The solvent was evaporated and then the antibody added in 0.25 ml of water (0.5 mg total protein). Iodine was then added and the reaction continued for 15 minutes with stirring at 10°C. The reaction was terminated by application of the reaction mixture to a prepacked Sephadex G50 column equilibrated with PBS. The solution was sterilized by filtration using a 0.22 micron filter.

2.2. Competitive solid phase radioimmunoassay

This was effected in order to examine whether the antibodies SWA11, -21 and -22 recognize the same epitope.

Target cells were fixed to 96 well plates. The plates were coated with poly-L-lysine and $5 \times 10^4$ cells fixed to each well using glutaraldehyde. Plates were stored at 3°C in PBS containing 1 % bovine serum albumin and 0.2 % sodium azide. Prior to use the plates were incubated with wash buffer for 30 minutes to prevent non-specific binding (Tris-buffered saline, 5 % non-fat milk and 1 % gelatine). Unlabelled antibodies SWA21 and SWA22, which show the same pattern of tissue reactivity as SWA11 were added at saturating concentration to the plate with SWA11 acting as positive control. Incubation was performed for 1 h at 37°C and then the plates washed 4x with PBS and 1 % bovine serum albumin. Radiolabelled SWA11 was then added at a concentration giving one half maximal binding in the absence of competition and further incubation performed at 37°C for one hour. After final washing (4x) individual wells were cut out and counted in a gamma counter.

The results are shown in Table II:

8

Table II

Competitive binding assay between SWA11,
SWA21 and SWA22

| Labelled antibody | Unlabelled antibody | % binding° |
|---|---|---|
| SWA11 | none | 100 |
| | SWA11 | 10.9 |
| | SWA21 | 11.1 |
| | SWA22 | 23.0 |

° expressed as percent binding of SWA11 in the
absence of competing antibody

Unlabelled SWA11 at saturating concentration reduced the binding of radiolabelled SWA11 to 10.9 % whereas SWA21 and SWA22 reduced binding to 11.1 and 23.0 % respectively. This is a further indication that the likelihood that the three monoclonals recognize the same epitope is very high.

2.3. In vitro immunoreactivity

To determine the biological activity of radiolabelled SWA11 SW2 cells were washed 3x in PBS (with 5 % non-fat milk, 0.05 % azide) and varying cell numbers were then incubated for 2 hours at 4°C with a fixed amount of radiolabelled antibody. After washing the activity in the cell pellet was counted. The number of counts remaining unbound was plotted against the reciprocal of the cell number.

The iodogen radiolabelling reagent was employed routinely to produce iodinated SWA11 with a specific activity in the range of 0.5 to 1.0 mCi/mg. The biological activity of the labelled antibody was generally 65-70 %. This figure was subsequently used in calculations relating to the number of antigens on SW2 cells and the $K_a$ of the interaction.

2.4. Binding affinity (determination of n and $K_a$)

The number of antigens, n, on the surface of SW2 cells capable of interaction with SWA11 and the association constant, $K_a$, were also assessed (M. Trucco & S. de Petris, Immunol. Methods, 2 [1981] 1). SW2 cells were washed 3x in PBS (with 5 % non-fat milk, 0.05 % azide) and then dispensed at $5 \times 10^4$ cells per tube. A serial two-fold dilution of radiolabelled SWA11 was performed and 100 µl added to each tube. The total reaction volume was 0.5 ml. The incubation was carried out at 4°C for 2 hours and then the cells washed 5x as above. The data obtained were manipulated to allow a plot of r against r/A-X to be made (where r is the number of antibody molecules bound per cell and A-X is the free antibody expressed as cpm). The interaction of SWA11 with the leukaemic cell line K562 and human peripheral blood buffy coat were also evaluated using the same technique in an attempt to estimate the significance of heterogenous staining of buffy coat and bone marrow observed by an indirect immunofluorescence test.

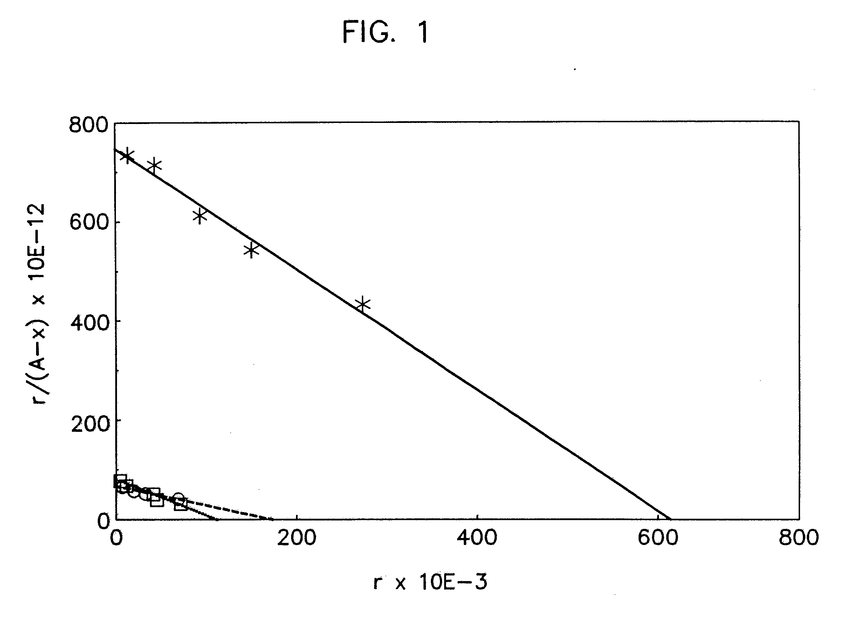

Data obtained from the interaction of SWA11 with the cell lines SW2 and K562 and with human peripheral blood buffy coat were plotted. From the intercepts on the y axis (= Kn) and the x axis (= n) the values of $K_a$ are easily obtained. In Figure 1 the numbers of antigenic sites, n, are equal to $6.1 \times 10^5$, $1.1 \times 10^5$ and $1.2 \times 10^5$ per cell for SW2, K562 and human buffy coat, respectively, showing that the antigen recognized by SWA11 is preferentially expressed on the SW2 cell line. The respective association constants, $K_a$, are $1.2 \times 10^9$, $6.8 \times 10^8$ and $5.6 \times 10^8$ $M^{-1}$, indicative of the high binding affinity and thus high potential as a localizing agent, particularly as regards SWA11.

**Example 3: In vivo characterization**

1. Tumor localization

SW2 xenografts

The SW2 small cell line was grown as subcutaneous xenografts in NMRI nu/nu mice. 2-3 $mm^3$ pieces of tumour were transplanted into 4-6 week old mice and within approximately three weeks the tumors were ready for use. All animals were maintained on pathogen-free food and acidified drinking water.

In vivo localization

In vivo distribution of SWA11 in the xenograft model system was determined by the simultaneous i.v. injection of $^{131}$I-labelled SWA11 and $^{125}$I-labelled anti-CEA control antibody. Both antibodies were of the IgG2a isotype. Mice were dissected at days 2, 3, 4 and 7 post injection and the various organs rinsed in PBS and then weighed and counted in a dual channel gamma counter. Localization of antibody was expressed in absolute terms as the percentage of injected dose per gram of tissue land in relative terms as a tissue-to-blood ratio.

9

Tissue-to-blood ratios of SWA11 and control anti-CEA at days 2, 3, 4 and 7 are presented graphically in Figure 2 a) and b). Whereas no selective localization of anti-CEA was observed, the tumour-to-blood ratios for SWA11 were 2.4:1, 5.3:1, 7.5:1 and 8.0:1 at days 2, 3, 4 and 7, respectively. At day 4 the level of tumour accumulated SWA11 was 19, 22 and 12 times higher than levels in liver, kidney and spleen, respectively. The absolute levels of SWA11 in tumour and various organs are presented in Table III. SWA11 displays a high localization at day 2 with 10.5 % injected dose/g of tumour. The antibody remains bound to the tumour, maintaining 8 % injected dose/g at days 3 and 4 and then falling to 4.8 % injected dose/g by day 7:

Table III

Tissue distribution of antibody SWA11°

| | Day | | | |
| | 2 | 3 | 4 | 7 |
|---|---|---|---|---|
| Tumour | 10.5 | 8.0 | 8.0 | 4.8 |
| Blood | 4.2 | 1.5 | 1.1 | 0.60 |
| Liver | 1.9 | 0.75 | 0.5 | 0.22 |
| Kidney | 1.4 | 0.7 | 0.37 | 0.12 |
| Spleen | 1.8 | 0.63 | 0.58 | 0.35 |
| Lung | 1.8 | 0.88 | 0.66 | 0.39 |
| Heart | 1.8 | 0.68 | 0.33 | 0.08 |
| Thyroid | 2.0 | 0.84 | 0.58 | 0.23 |
| Femur | 1.0 | 0.36 | 0.25 | 0.07 |
| Muscle | 0.8 | 0.35 | 0.18 | 0.04 |
| Brain | 0.25 | 0.10 | 0.05 | 0.013 |

° Expressed as percent total injected dose per g tissue

## 2. Gamma scintigraphy

Xenografted mice were injected i.v. with 100 $\mu$Ci of $^{131}$I-labelled SWA11 (100 $\mu$Ci/100$\mu$g). Imaging was performed on days 2 and 4 using a pinhole collimator positioned 9 cm from the target animal and then linked to a Picker Dyna Camera 4. Each image was generated from 50 000 counts acquired using an energy window of 25 % centered around 364 keV. Background substraction and thyroid blocking were not employed. Animals were anaesthetised during imaging by the intra peritoneal injection of 0.5 ml Nembutal (1:10 dilution in PBS).

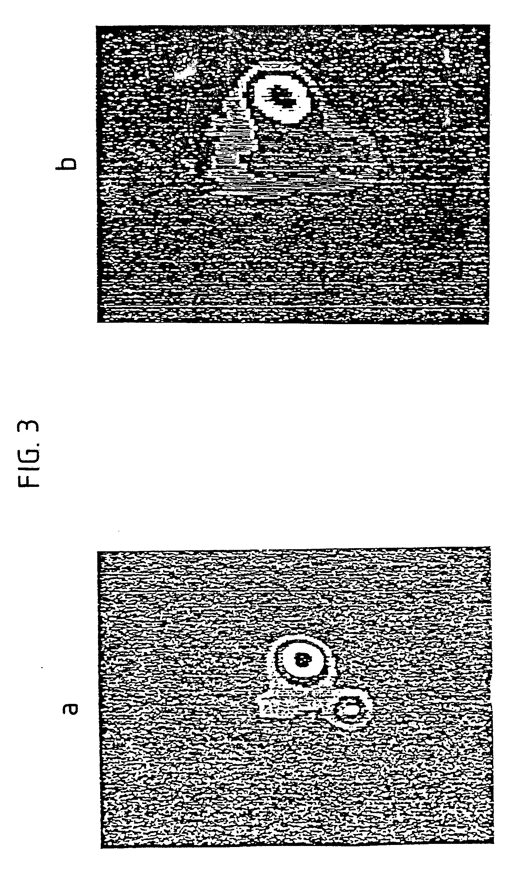

The selective accumulation of SWA11 in SW2 xenografts at 2 and 4 days following injection was confirmed by external gamma scintigraphy as displayed in Figure 3 a) and b), respectively. At day 2 the tumour was already clearly visible against the background of activity still present in the blood pool in the visceral organs of the thorax. By day 4 the background level was markedly reduced whereas the tumour remained relatively constant in terms of image intensity. The unblocked thyroid was visible at this time due to accumulation and relative retention of free [131]I.

Taken together the above results are indicative of an exceptional ability of SWA11 to localize to SCC xenografts.

## Example 4: Radioimmunotherapy of SCC xenografts using $^{131}$I-SWA11

Radioimmunotherapy of SCC xenografts was performed by i.v. injection of 300 $\mu$Ci of $^{131}$I-labelled SWA11 into nude mice bearing established tumors in the 0.5 - 1.0 cm$^3$ range. Animal weight and tumor diameter in two dimentions were recorded on day 0 and were subsequently monitored. The tumor volumes were calculated using the formula

$$v = \frac{d^3 \, \pi}{6}$$

wherein v = tumor volume
d = mean tumor diameter

A significant effect on tumor volume was evident from approximately day 4 post injection. Controls included SWA11 antibody alone, 300 $\mu$Ci of $^{131}$I-labelled irrelevant antibody and untreated animals.

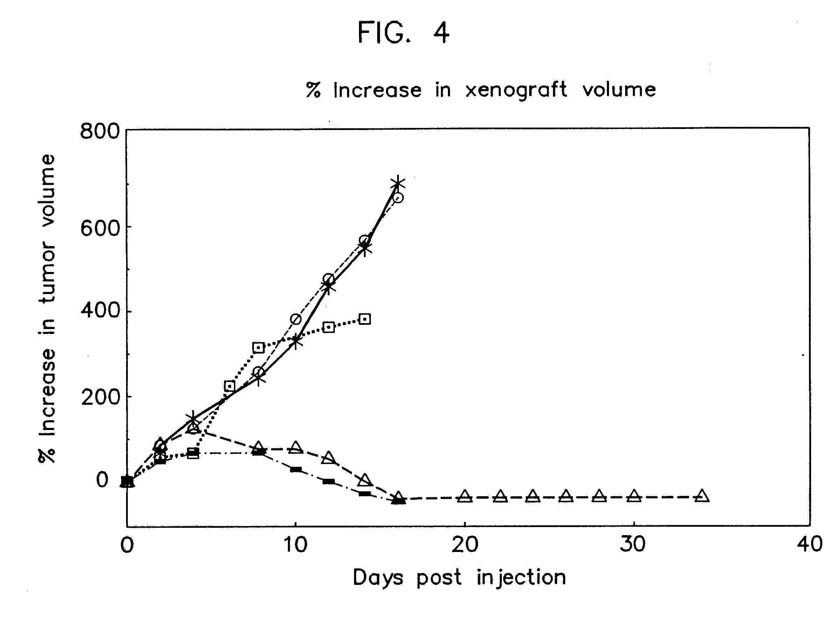

The results are shown in Figure 4. The beneficial effect with SWA11 lasts for several weeks, with 300 $\mu$Ci it lasts for more than at least a month.

Tumor histology:
   At 16 days post injection control animals were killed because of excessive tumor burden. Two animals treated with $^{131}$I-SWA11 were also killed at this time point to facilitate a direct comparaison of tumor histology. Tumor samples were fixed in 4 % formaldehyde and then sectioned and stained.

Untreated tumors:
These appeared to be very vigorously growing tumors of characteristic SCC morphology. Large areas of densely packed small cells of polymorphic character were present, as were substantial areas of necrosis as would be expected of such large tumors.

Tumors treated with radiolabelled control antibody:
These were generally similar to untreated tumors with large areas of densely packed and rapidly dividing small cells and regions of necrosis. Some evidence of radiation damage was present however in the form of large cells of malignant origin which were presumably blocked in cell division.

Tumors treated with radiolabelled SWA11:
These tumors were almost completely necrotic with only a very limited number of malignant cells remaining, all of which very large and representing a population of unknown clonogenic potential. There was abundant proliferating fibrotic tissue of murine origin.

## Example 5: Detection and characterization of antigen

   1. Extraction of antigen from SW2 cell line. For Western blot analysis, cells were washed once in PBS (phosphate-buffer saline) and solubilised in 0.2 % N-dodecyl-N,N-dimethylammonio-3-propanesulfonate, 1 mM phenylmethyl sulfonyl fluoride, 0.1 mM leupeptine and 50 μg/ml pepstatin A in ice-cold PBS for 30 minutes. After centrifugation for one hour at 100000 g at 4°C, the supernatant was heated 30 minutes at 97°C, centrifuged as before and the supernatant was used for the Western blot analysis of SWA11 antigen.

   2. Antigens transferred from SDS-gels to nitrocellulose. Electrophoresis was accomplished on 12.5 % or 15 % SDS-polyacrylamide gels under reducing conditions using the buffer system of O'Farrel. Aliquots of $10^7$ cells were transferred electrophoretically onto 0.2 μm nitrocellulose according to the method of Otter (Anal. Biochem. 162 [1987] 370). Before transfer, antigens were renatured by incubating the gels for 20 minutes in 6M urea, 20 minutes in 3M urea and 20 minutes in transfer buffer. The electrotransfer was accomplished in a two step procedure, beginning with the elution of low-weight molecules at low current density (1 mA/cm²) for one hour, followed by prolonged electrotransfer (20 hours) at high current density in transfer buffer (49 mM Tris, 384 mM glycine, 20 % methanol (v/v), and 0.01 N-docecyl-N,N-dimethylammonio-3-propanesulfonate. After electrotransfer the nitrocellulose sheet was oven-dried (100°C/15 minutes) and quenched by incubation (30 minutes) in Tris buffered saline (TBS: 25 mM Tris; 0.5 M NaCl; pH 8) containing 5 % non-fat dried milk. The sheet was then incubated for 24 hours at 4°C with continuous rotation with SWA11. After washing (3 times 10 minutes) with the quenching buffer and 0.05 % Tween 20 the sheet was incubated with affinity-purified alkaline phosphatase goat anti-mouse IgG diluted 1:3000 in PBS and 10 % normal goat serum, pH 6.5 for one hour. After washing as before, but at pH 6.5, alkaline phosphatase was demonstrated at 50 mM ethanolamine, 1.0 mM MgCl₂, pH 9.8 using BCIP/NBT (5-bromo-4-chloro-3-indolyl phosphate p-toluidine / Nitrotetrazolinum Blue chloride) as the color development solution.

   3. Antigen on thin layer chromatograms. Glycolipids were isolated from SW2 cells as follows. Cell pellets ($10^7$ cells) were washed twice with PBS and lyophilised. The lipids were extracted using chloroform and methanol (2:1 by volume) with three times 2 minutes sonication. The extract was filtered through a 0.22 μM filter and evaporated on a Speed-Vac concentrator. Crude lipids were chromatographed on high performance thin layer chromatography silica plates in chloroform: methanol: 0.25 % KCl (5:4:1). After air-drying, the plates were either developed with ninhydrin spray reagent or immunostained as follows: the chromatograms were soaked for two hours at room temperature in PBS and 5 % non-fat dry milk to quench non-specific binding. The plates were incubated overnight in SWA11 supernatant, washed twice 30 minutes as before and incubated with affinity-purified peroxidase-conjugated goat anti-mouse IgG diluted 1:500 in PBS and 10 % horse serum for 2 hours. After washing as before peroxidase activity was demonstrated using 4-chloro-1-naphthol as substrate.

   4. Immunofluorescence on suspension of SW2 cells. SW2 cells were either grown in RPMI ((Roosevelt Park Memorial Institute) medium and 10 % FCS (fetal calf serum) or RPMI medium, 10 μg/ml tunicamycin and 10 % FCS. For screening by indirect immunofluorescence, cells were washed with PBS, 0.1 % sodium azide and 10 % horse serum. The cells were aliquoted in tubes and centrifuged at 800 g for 5 minutes. After aspirating out the supernatant, 100 μl of SWA11, SWA21, SWA22, SWA20 or LAM8 were applied. The tubes were lightly vortexed and incubated for 1 hour at 37°C. The cells were washed twice with PBS and 10 % horse serum, the supernatant aspirated out and 30 μl of the second antibody (1:20 dilution of FITC (fluoresceine-isothiocyanate)-labeled goat antimouse Ig) applied. The tubes were lightly vortexed and incubated for 1 hour at 37°C. After washing as before, the cells were added to a slide

11

covered with a cover slip. The antibody reactivity was observed with green fluorescence on a Leitz microscope.

5. Radioimmunoassays. SW2 cells were bound to 96-well plates as target for radioimmunoassays (C.H. Heusser et al., Methods Enzymol. 73 [1981] 407). The wells were coated with poly-L-lysine and $5\times10^4$ cells were fixed with glutaraldehyde to each well. The plates were stored at 4°C with 1 % BSA and 0.2 % sodium azide in PBS. For competitive inhibition of antibody binding to target cells, the optimum concentration of antibody was titrated using one-half of the the maximal binding concentration. To test competition with lectins, target plates were preincubated with 0.1 mg lectin/well for 1 hour in PBS, washed twice in PBS and 1 % BSA and incubated with the respective amount of antibody in 100 μl PBS and 1 % BSA. To test the influence of enzymes, wells were incubated with 1 unit enzyme at 37°C for 1 hour, washed twice in PBS and 1 % BSA and incubated with the respective amount of antibody in 100 μl PBS and 1 % BSA. For periodate treatment of the antigens, wells were incubated with 20 mM periodate for 15 minutes at room temperature, the reaction stopped with ethylene glycol, the wells washed twice in PBS and 1 % BSA and incubated with the respective amount of antibody in 100 μl PBS and 1 % BSA.

## Table IV

Characterization of the epitope recognised by antibodies SWA11, SWA21 and SWA22 using solid phase-RIA on SW2 target cells pre-treated with lectins, enzymes or periodate. The results are expressed as percent binding on small cell carcinoma cell line compared with SWA20

|  | SWA11 | SWA21 | SWA22 | SWA20* |
|---|---|---|---|---|
| Enzyme treatment | | | | |
| Neuraminidase (1 unit) | 90 | 105 | 106 | 11 |
| Trypsin (1 unit) | 75 | 50 | 65 | 75 |
| Chymotrypsin (1 unit) | 34 | 25 | 25 | 7 |
| Lectin preincubation | | | | |
| Solanum tuberosum | 60 | 60 | 70 | 54 |
| Triticum vulgaris | 15 | 16 | 18 | 26 |
| Periodate treatment (20 mM) | 80 | 112 | 120 | 65 |

* EP 323802

## Table V

Characterization of the epitope recognized by the antibodies SWA11, SWA21, SWA22 and SWA20 and LAM8 by indirect immunofluorescence on live SW2 cells with cells grown in normal medium and cells grown in medium supplemented with 10 µg/ml tunicamycin. The results are expressed in fluorescence intensity (1= weak; 4= strong).

|  | SWA11 | SWA21 | SWA22 | SWA20*/LAM8 |
|---|---|---|---|---|
| Normal SW2 cells | +4 | +4 | +4 | +3 |
| (homogen) | (homogen) | (homogen) | (heterogen) | |
| Tunicamycin-treated cells | +4 | +4 | +4 | 0 |

\* EP 323802

## Claims

1. A monoclonal antibody or functional fragment thereof specific for a tumor-associated cell surface membrane-expressed antigen of the small cell carcinoma of the lung characterised in that it
- belongs to Cluster w4 as defined at the International Workshop on SCC, London 1987,
- shows high capacity for in vivo localisation,
- has low retention in non-target organs,
- exhibits persistence at the target tumor and
- binds to a glycoprotein antigen having the following characteristics:
- molecular weight of approximately 45000 before and about 15000 to about 17000 after deglycosylation;
- remains reactive with antibody SWA11 after deglycosylation;
- remains present in cells after treatment with tunicamycin.

2. An antibody according to claim 1 which is of IgG isotype.

3. An antibody according to claim 1 which is produced by the hybridoma cell line ECACC 88080301.

4. An antibody according to claim 1 which is coupled to a cytotoxic agent.

5. A hybridoma cell line capable of producing a monoclonal antibody as defined in claim 1.

6. The cell line according to claim 7 which is ECACC 88080301.

7. A tumor-associated antigen selectively expressed on the cell surface membrane of a proportion of small cell carcinoma of the lung characterized in that it is a glycoprotein which
- has a molecular weight of approximately 45000 before and about 15000 to about 17000 after deglycosylation;
- remains reactive with antibody SWA11 after deglycosylation and
- remains present in cells after treatment with tunicamycin.

8. A pharmaceutical composition comprising an amount of a monoclonal antibody as defined in claim 1 sufficient to suppress small cell carcinoma of the lung, and a pharmaceutical carrier therefor.

9. A monoclonal antibody according to any one of claims 1 to 4 for use as a pharmaceutical.

10. A method of suppressing small cell carcinoma of the lung which comprises administering to a subject in need of such treatment a therapeutically effective amount of a monoclonal antibody as defined in claim 1.

## Claims for the following Contracting States: GR, ES

1. A process for the production of a monoclonal antibody specific for a tumor-associated cell surface membrane-expressed antigen of the small cell carcinoma of the lung which
- belongs to Cluster w4 as defined at the International Workshop on SCC, London 1987,
- shows high capacity for in vivo localisation,
- has low retention in non-target organs,
- exhibits persistence at the target tumor and
- binds to a glycoprotein antigen having the following characteristics:
- molecular weight of approximately 45000 before and about 15000 to about 17000 after deglycosylation;
- remains reactive with antibody SWA11 after deglycosylation;
- remains present in cells after treatment with tunicamycin,
comprising growing a corresponding hybridoma cell line and isolating the monoclonal antibody therefrom.

2. A process according to claim 1 for the production of a monoclonal antibody as defined in claim 1 which is of IgG isotype.

3. A process according to claim 1 for the production of a monoclonal antibody as defined in claim 1 which is produced by the hybridoma cell line ECACC 88080301.

4. A process according to claim 1 for the production of a monoclonal antibody as defined in claim 1 which is coupled to a cytotoxic agent.

5. A process for the production of a hybridoma cell line capable of producing a monoclonal antibody as defined in claim 1 which comprises fusing an appropriate myeloma cell with an appropriate antibody-producing lymphocyte.

6. A process according to claim 5 wherein the cell line is ECACC 88080301.

7. A process for the preparation of a pharmaceutical composition comprising mixing a monoclonal antibody as defined in claim 1 with a pharmaceutical carrier therefor.

8. A method of detecting small cell carcinoma of the lung which comprises contacting a source suspected of having SCC of the lung with a diagnostically effective amount of a detectably labelled monoclonal antibody as defined in claim 1 and determining whether said antibody binds to said source.

9. The method according to claim 8 wherein said antibody is produced by hybridoma cell line ECACC 88080301.

FIG. 1

FIG. 2

FIG. 3

a

b

FIG. 4

# FIG. 5

SWA-11 specific antigens